Europäisches Patentamt

(19) European Patent Office

Office européen des brevets

(11) Numéro de publication: **0 358 585**

**A1**

(12) **DEMANDE DE BREVET EUROPEEN**

(21) Numéro de dépôt: **89420331.4**

(22) Date de dépôt: **08.09.89**

(51) Int. Cl.5: **C 08 L 89/06**
**A 61 K 35/36, A 61 K 37/12**

(30) Priorité: **09.09.88 FR 8812061**
**09.09.88 FR 8812062**

(43) Date de publication de la demande:
**14.03.90 Bulletin 90/11**

(84) Etats contractants désignés:
**AT BE CH DE ES FR GB GR IT LI LU NL SE**

(71) Demandeur: **CENTRE TECHNIQUE CUIR CHAUSSURE MAROQUINERIE**
**4 rue Hermann Frenkel**
**F-69007 Lyon (FR)**

(72) Inventeur: **Vulliermet, Arlette**
**25 A rue de Montribloud**
**F-69009 Lyon (FR)**

**Sanejouand, Jérôme-Henri**
**8 rue Colette**
**F-69008 Lyon (FR)**

(74) Mandataire: **Guerre, Dominique et al**
**Cabinet Germain et Maureau 20 Boulevard Eugéne**
**Deruelie BP 3011**
**F-69392 Lyon Cédex 03 (FR)**

(54) **Procédé d'obtention de collagène natif à partir de peaux animales.**

(57) Le procédé selon l'invention consiste à défibriller complètement le tissu dermique des peaux par voie exclusivement mécanique sans adjonction de produit chimiquement actif, ces peaux ayant été préalablement lavées avec une solution aqueuse froide, et refendues de manière à obtenir des parties derme, d'une part, et des parties fleurs, d'autre part.

Application du collagène obtenu notamment dans les domaines de l'alimentation humaine et animale, de l'emballage alimentaire, de l'hygiène corporelle, de la pharmacie humaine et animale, de la cosmétique et de l'industrie textile et papetière.

EP 0 358 585 A1

**Description**

## PROCEDE D'OBTENTION DE COLLAGENE NATIF A PARTIR DE PEAUX ANIMALES

La présente invention concerne un procédé d'obtention de collagène natif, c'est-à-dire non dénaturé, à partir de peaux animales.

Comme on le sait, le collagène est une protéine animale déjà utilisée dans de nombreuses applications et, en particulier, comme ingrédient alimentaire pour ses propriétés de liant, de rétenteur d'eau, de texturant, de fixateur d'arômes et de colorants, de gélifiant, de solubilisant et d'agent nutritif. Le collagène sous forme de gel peut servir de base à la fabrication d'emballages alimentaires comestibles comme les enveloppes de saucisses. On constate, à l'heure actuelle, que les besoins en cette matière première ne vont qu'en s'accroissant.

Les peaux résultant de l'abattage de différentes espèces animales constituent une source essentielle et privilégiée de collagène, à la condition que les différents traitements ou opérations affectant la peau, au moment de l'abattage ou postérieurement à ce dernier, n'affectent pas l'intégrité de la structure collagénique.

Conformément au brevet FR-A-1 568 829, on a déjà proposé d'obtenir une pâte, dispersion ou gel collagénique, à partir de peaux animales, selon le procédé suivant :
- lavage éventuel de la peau traitée
- broyage direct et fin de la peau, avec refroidissement, par exemple avec un hâchoir, pour obtenir un broyat
- essorage éventuel du broyat
- malaxage du broyat, en présence d'acide chloracétique concentré, toujours à froid ou avec refroidissement, pour obtenir une pâte hétérogène
- dilution du broyat avec une solution d'acide chloracétique dilué, toujours à froid, pour obtenir finalement une dispersion collagénique, relativement pauvre en matière sèche et donc en collagène.

Un tel procédé présente les inconvénients suivants.

Il ne préserve pas suffisamment, c'est-à-dire pour autant qu'il est souhaitable dans les différentes applications du collagène natif, la structure et les propriétés physico-chimiques de ce dernier, pour plusieurs raisons :
- le broyage direct à l'état fin des peaux est une opération générant beaucoup de chaleur, du fait de la cohésion et donc de la résistance mécanique importante du feutre dermique ; elle est donc difficile à contrôler en température, et des surchauffes locales de la charge traitée existent nécessairement ; tout excès de température affecte et dégrade la structure physico-chimique du collagène
- l'acidification participant au procédé affecte nécessairement l'intégrité de la structure macromoléculaire du collagène natif.

Il conduit à un rendement limité en collagène utilisable, c'est-à-dire non dénaturé.

La présente invention a pour objet un procédé de traitement complet permettant de récupérer presque complètement et à l'état non dénaturé, le collagène natif présent dans les peaux traitées.

Selon l'invention, les matières premières utilisées ressortent de deux catégories.

Tout d'abord, les peaux de départ, traitées directement pour obtention du collagène, peuvent être des peaux brutes ou fraîches. Ces peaux fraîches, non retenues pour diverses raisons, représentent une source de collagène natif tout à fait significative, et peuvent être valorisées selon le procédé conforme à l'invention. Par "peau frâiche", on entend une peau provenant directement de l'abattoir, après la dépouille de l'animal.

Ensuite, les peaux de départ, retenues pour traitement selon la présente invention, peuvent être dans un état d'élaboration intermédiaire entre l'état brut ou frais précité, et l'état complètement tanné, c'est-à-dire à l'état du cuir, pour autant que dans cet état intermédiaire le collagène constitutif ait conservé pour l'essentiel son intégrité.

A titre d'exemple non limitatif de cet état intermédiaire, la peau de départ peut être une peau préconditionnée, c'est-à-dire n'ayant subi aucun traitement chimique irréversible, en particulier non tannée, et biologiquement stabilisée. Il s'agit de peaux ayant subi diverses opérations leur permettant de se conserver de façon satisfaisante pendant des durées relativement longues. Ces opérations sont les premières étapes de la chaîne de transformation des peaux fraîches en cuir, à savoir : travail de rivière, écharnage, déchaulage, confitage ainsi qu'un préconditionnement du type de ceux consistant à un picklage et à un prétannage, en vue d'obtenir des peaux préconditionnées dites Blanc Stabilisé Humide (BSH) ou des peaux dites Blanc Stabilisé Sec (BSS).

Un procédé permettant d'obtenir des peaux dites BSH est notamment décrit dans l'article suivant, dans la revue suivante :
- LEDER, Vol 36, N° 10 - 1985 - pages 170-175 - DARMSTADT - DE ; L.TONIGOLD ET AL - Herstellung von Wet White durch eine Aluminium gerbung und deren anschliessende Weiterverarbeitung.

Quant au procédé permettant d'obtenir des peaux dites BSS, il est notamment décrit dans le brevet FR-C-2 610 643.

Le contenu de ces deux documents est intégré en tant que de besoin à la présente description.

L'état de présentation des peaux préconditionnées améliore les possibilités d'appréciation de la qualité du matériau par les spécialistes, de façon à permettre une meilleure orientation des peaux en fonction de leur qualité et des besoins du marché. A ce stade, elles n'ont pas subi de traitements chimiques irréversibles, susceptibles d'endommager la structure du collagène dermique et, par là même, d'amoindrir ses propriétés. Il est donc possible de revaloriser les peaux de très bas choix inutilisables pour la production de cuir, en les inégrant dans un circuit de fabrication de collagène, selon la présente invention.

Selon l'invention, on procède à une défibrillation quasi-complète de la peau traitée, sans adjonction de produit chimiquement actif vis-à-vis du collagène natif, au moins par les opérations successives suivantes :

a) éventuellement lavage des peaux traitées avec une solution aqueuse froide

b) refendage de la peau lavée, pour obtenir une partie derme d'une part, et une partie fleur d'autre part

c) réduction ou division mécanique progressive de la surface, et à froid, de la partie fleur et/ou de la partie derme, par prédécoupage en pièces de surface réduite, puis découpage desdites pièces en morceaux plus petits, en présence d'un agent de refroidissement et de dilution, prévu pour amener la matière sèche de la suspension obtenue à une concentration comprise entre 10 et 30 % en poids, et maintenir la température inférieure à environ 40°C

d) traitement postérieur par broyage, par voie sèche ou humide, de la ou des suspensions obtenues, du type hâchis, pour détruire le feutrage dermique, et obtenir au moins un produit final riche en collagène fibreux pratiquement non dénaturé.

Comme déjà dit précédemment, la peau de départ peut être une peau brute ou fraîche, moyennant quoi la peau traitée selon le procédé précédent s'identifie à la peau brute ou fraîche.

Mais aussi, selon l'invention, la peau de départ peut être une peau dans un état intermédiaire, notamment préconditionné, par exemple une peau dite BSS ou BSH. Dans ce cas, la peau traitée est obtenue par déconditionnement de la peau de départ, avant, ou en cours, par exemple au début du procédé selon l'invention. Ce déconditionnement s'effectue en particulier avec des agents neutralisants et bactéricides.

L'opération chimique de décontionnement a une action spécifique limitée à la destruction des liaisons entre le collagène et les produits de préconditionnement ayant permis la stabilisation et la conservation des peaux. Il s'agit notamment de l'aluminium dans le cas des peaux dites B.S.H ou des peaux dites B.S.S. Ce traitement ne dénature pas le collagène du derme ; celui-ci conserve son arrangement en fibres constituées par des triples hélices. Seules certaines protéines, comme l'élastine, composant le tissu interfibrillaire du derme ont disparu sous l'effet du préconditionnement.

Avantageusement, le traitement de déconditionnement consiste à mettre au moins une fois les peaux en agitation dans un bain d'une solution aqueuse d'acide et de sel, à les laver au moins une fois sous agitation à l'aide d'un bain d'une solution saline aqueuse, et à les neutraliser sous agitation en présence d'une solution saline et alcaline.

De préférence, le déconditionnement intervient au début du procédé selon l'invention, après refendage, mais avant réduction ou division mecanique progressive de la surface de la peau.

Par voie humide selon l'invention, la division méanique progressive est effectuée sur la partie derme, pour obtenir une suspension comprenant entre 10 et 30 % de matière sèche. Le traitement postérieur est alors effectué sur le mélange ou suspension ainsi obtenu, par broyage progressif et à froid (température au plus égale à 40°C) de ce dernier, toujours en présence d'un agent de refroidissement et de dilution, pour obtenir en final une pâte fine et humide de collagène.

Par voie sèche selon l'invention, la division mécanique progressive est effectuée sur la partie fleur, et le traitement postérieur est effectué sur la suspension ou hâchis obtenu par cette division de la partie derme. Ce traitement postérieur consiste en un sèchage, éventuellement sous vide partiel, à une température au plus égale à 35°C, de la suspension, pour obtenir un granulat sec, puis en une réduction par broyage du granulat, pour obtenir finalement une poudre fine de fibres de collagène. Ces dernières peuvent avoir une longueur au plus égale à 0,5 mm. La poudre ainsi obtenue peut avoir une teneur en eau comprise entre 15 et 25 % en poids.

La présente invention permet de préserver l'édifice hélicoïdal et tridimensionnel du collagène, par la grande progressivité du procédé utilisé, non montrée ou suggérée par l'art antérieur, cette progressivité résidant dans :
- le refendage de la peau, et le traitement postérieur et séparé du derme et de la fleur
- la division mécanique de la surface du derme et/ou de la fleur, avant la destruction du feutrage dermique
- la division mecanique elle-même effectuée de manière progressive.

Cette progressivité jointe à un refroissement efficace permet de récupérer le collagène natif, sans adjonction de produit chimique, et en particulier sans modification de pH, et donc par voie exclusivement mécanique. Il faut noter également l'absence de tout antiseptique pouvant nuire à la qualité du collagène.

Au total, on obtient un collagène sous toute forme appropriée vis-à-vis de son utilisation ultérieure, ayant conservé toutes ses propriétés, organoleptiques en particulier.

Grâce à l'invention, et dans le cas des peaux brutes ou fraîches, les autres entités moléculaires composant le derme, telles que les glycosaminoglycanes ou l'élastine sont également préservées intactes.

Selon la présente invention, et de préférence, l'agent de refroidissement et de dilution est constitué par de l'eau froide, ou de la glace en paillettes.

La composition chimique de la pâte fine et humide, pouvant être obtenue par voie humide selon l'invention, à partir de peaux fraîches, est la suivante :
pH : 6,5 0,5
Matières sèches : 15 à 18 % en poids dont 94 % minimum de collagène fibreux
Matières grasses : inférieur à 0,5 % en poids
Matières minérales : inférieur à 0,1 % en poids

La composition chimique de la pâte fine et humide, pouvant être obtenue par voie humide selon l'invention, à partir de peaux préconditionnées, est la suivante :
pH : 6,2 0,3
Matières sèches : 15 à 20 % en poids, dont au moins 85 % de collagène
Matières grasses : inférieur à 1 % en poids
Matières minérales : inférieur à 1,5 % en poids
Aluminium : inférieur à 30 mg/kg

La pâte fine et humide peut être soit utilisée directement comme ingrédient alimentaire après avoir été éventuellement conservée au froid, soit soumise à un gonflement acide puis combinée à des liants et/ou plastifiants pour ensuite être transformée en film d'emballage alimentaire par exemple par extrusion, enrobage ou autre technique de recouvrement, soit désydratée et utilisée de façon nouvelle et originale sous la forme de fibres sèches de grande longueur comprise entre 1 et 2 centimètres, en complément ou non de fibres cellulosiques, notamment pour la confection de divers produits d'hygiène corporelle tels que les tampons ou serviettes périodiques, les couches pour bébés ou similaires, de produits pharmaceutiques pour la médecine humaine ou vétérinaire tels que les pansements hémostatiques ou similaires, ou bien encore de produits cosmétiques tels que les masques de beauté ou autres compositions.

Par voie sèche selon l'invention, pour des peaux brutes ou fraîches, de manière préférentielle mais non exclusive, entre le refendage et la division mécanique progressive des parties fleur, ces dernières sont épilées très rapidement chimiquement, afin d'éliminer les poils et l'épiderme, puis ensuite neutralisées, et lavées à l'eau froide. Il faut insister sur le fait que ce traitement chimique d'épilation a un effet ciblé et localisé aux follicules pileux et à l'épiderme. Par conséquent, il ne saurait endommager en aucune manière le collagène du derme.

La présente invention est maintenant décrite par référence aux deux diagrammes en annexe, dont le contenu est incorporé à la présente description :

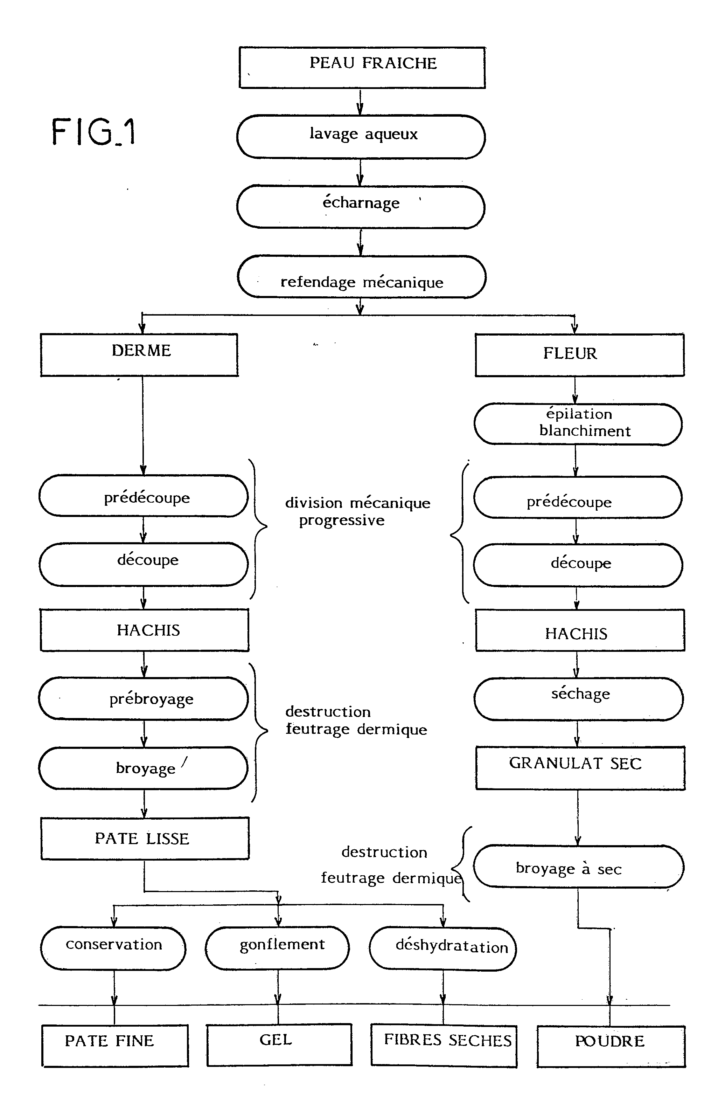

    - le premier diagramme (figure 1) schématisant le procédé selon l'invention, à partir de peaux sèches

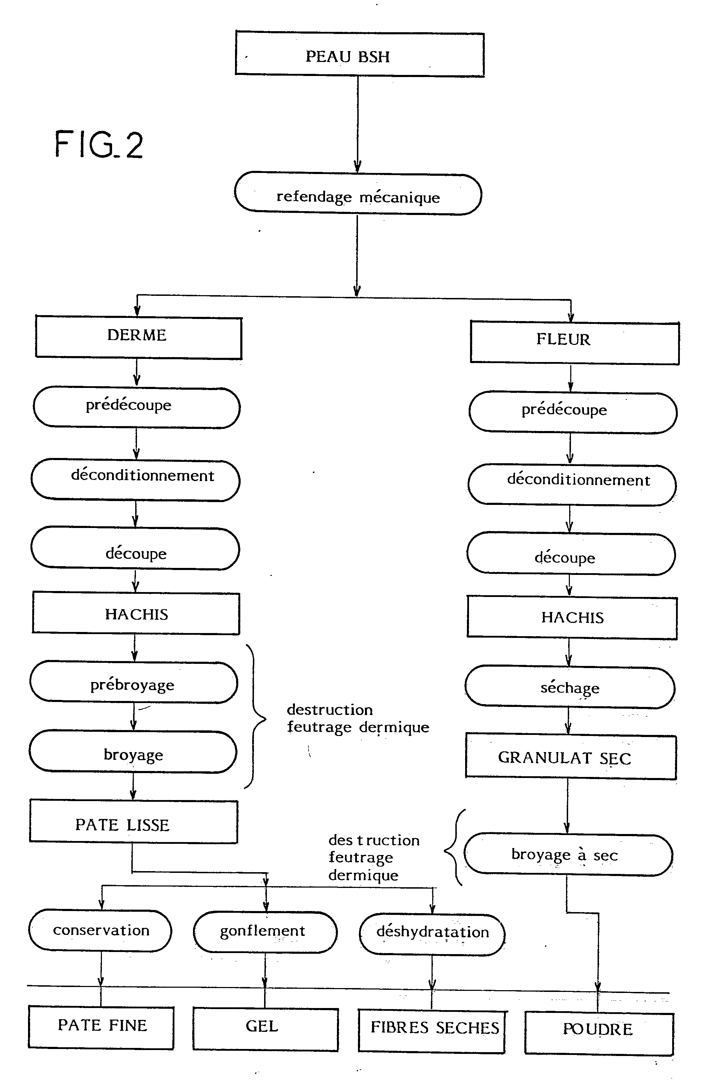

    - le second (figure 2) schématisant le procédé selon l'invention, à partir de peaux préconditionnées dites BSH.

## TRAITEMENT A PARTIR DE PEAUX FRAICHES

Les peaux fraîches utilisées peuvent être de toute nature : bovine, ovine, caprine ou autres.

On part de 1 000 kilogrammes de peaux de bovins fraîches qui sont, tout d'abord, lavées au foulon avec 3 000 litres d'eau froide. Après trente à soixante minutes de rotation, le bain de lavage est évacué et les peaux sont sorties du foulon. Elles sont alors écharnées de façon à éliminer le tissu sous cutané, et refendues en épaisseur à l'aide de machines identiques à celles utilisées en tannerie.

On obtient 850 kilogrammes de peaux écharnées qui donnent, après refendage, 340 kilogrammes (1/3) de parties fleurs et 510 kilogrammes de parties dermes (2/3).

Conformément à l'invention, les parties dermes sont destinées à la fabrication de pâte humide à base de collagène, tandis que les parties fleurs sont destinées à la fabrication de poudre sèche de collagène.

### Fabrication de pâte humide (derme)

Dans le cadre de la fabrication de pâte humide, le procédé consiste à défibriller complètement le tissu dermique par voie exclusivement mécanique. La texture du derme, composé de faisceaux de fibres de collagène entrelacées en un réseau tridimensionnel, lui confère une résistance mécanique élevée. Si l'on tente de détruire cette texture trop brutalement par une opération mécanique quelconque, la résistance du tissu dermique se traduit par une élévation de température d'autant plus forte que cette action est violente. Or, en ce qui concerne le collagène, il est bien connu qu'à partir de 40°C commence une dissociation moléculaire conduisant à un effondrement de l'édifice et à une dénaturation du collagène lui-même.

Le traitement mécanique consiste donc, tout d'abord, en une réduction ou division progressive de la surface du derme, à défibriller, à l'aide de différents types de dispositifs de coupe, suivie d'une défibrillation complète par destruction du feutrage dermique par voie mécanique également.

Les 510 kilogrammes de dermes sont, tout d'abord, prédécoupés grossièrement en pièces d'environ 20 x 20 centimètres dans une coupeuse à couteaux circulaires du type de celle commercialisée par la Société "Laursen". Le dispositif de coupe doit allier bon tranchant et maintien de le peau en tension ou en pression.

Les pièces ainsi prédécoupées sont ensuite réduites ou découpées en petits morceaux d'environ 3 x 3 centimètres, dans une machine du type de celle commercialisée sous la dénomination "Cutter" 130 l vertical "Stephan" ,(société domiciliée en RFA), en mélange avec 383 kilogrammes de glace en paillettes, afin d'amener la matière sèche à une concentration d'environ 20 % en poids.

Le mélange ou hâchis obtenu alimente un pré-broyeur du type de celui commercialisé sous la dénomination "Microcut" par la Société "Stephan", dont l'organe de coupe est constitué d'une tête à six dents et d'une couronne avec lames inclinées à dix-neuf dents. Cette opération permet d'obtenir 893 kilogrammes d'une pâte très grossière destinée à alimenter le broyeur proprement dit. A ce stade, le produit obtenu est composé de tous petits morceaux de peaux de quelques millimètres mais dont chacun a conservé sa structure primitive, à savoir le réseau tridimensionnel de fibres de collagène. La dernière étape de broyage va donc consister à faire éclater cette structure.

Afin de réaliser la phase finale sans une élévation de température au delà de 40°C, il est nécessaire d'ajouter encore 233 kilogrammes de glace en paillettes ou d'eau froide aux 893 kilogrammes précédents, pour obtenir 1 116 kilogrammes de pâte fine et humide. Le dispositif de broyage est formé par un couple rotor/stator, équipé chacun de séries de couronnes concentriques crénelées de lames tranchantes avec des inclinaisons différentes, et d'un système de répartition à l'entrée de l'appareil. Un broyeur de ce type est commercialisé sous la dénomination "Trigonal" par la Société Siefer (domiciliée en RFA). La pâte fine et humide ainsi obtenue peut être soit utilisée immédiatement, soit conservée par le froid.

Cette pâte est donc constituée d'eau et de fibres de collagène bien différenciées. On le vérifie facilement au microscope. Sa composition chimique est la suivante :

pH : 6,5 ± 0,3
Matières sèches : 16 - 18 % en poids dont 94 % minimum de collagène
Matières grasses : inférieur à 0,5 % en poids
Matières minérales : inférieur à 0,1 en poids.

Le procédé selon l'invention permet le passage direct du collagène présent à l'état natif dans le derme à un collagène à l'état de fibres dans la pâte fine et humide, sans dégradation de sa structure et avec conservation de toutes ses propriétés intéressantes. La pâte fine et humide présente l'avantage de pouvoir être conservée et/ou employée directement comme ingrédient alimentaire par exemple dans la filière viande du fait des propriétés fonctionnelles du collagène qui peuvent influer sur la valeur esthétique et les propriétés organoleptiques des aliments, tandis que sa digestibilité, sa disponibilité biologique, sa composition en acides aminés apportent un complément nutritionnel important.

Elle peut être également traitée par gonflement acide pour être transformée en gel apte à constituer en complément avec des liants et plastifiants connus en soi des emballages alimentaires fabriqués par extrusion, ou autres techniques de recouvrement.

En outre, elle peut être déshydratée et les fibres sèches et longues récupérées peuvent notamment servir de base à la fabrication de pansements hémostatiques, de tampons hygiéniques, de couches pour bébés ou similaires.

D'une façon générale, la pâte fine et humide, le gel et les fibres sèches de collagène obtenus par le procédé selon l'invention peuvent être utilisés comme ingrédient dans le domaine de l'alimentation humaine et animale, dans le domaine des produits d'hygiène corporelle, pharmaceutiques pour la médecine humaine ou vétérinaire, cosmétiques, ou dans le domaine de l'industrie textile et papetière pour être employés dans la confection de matériaux tissés ou non.

Comme indiqué précédemment, les parties fleurs obtenues après refendage mécanique permettent la fabrication de poudre sèche de collagène.

### Fabrication de poudre sèche (fleur)

Selon une opération connue en soi et classique en tannerie, les poils et l'épiderme des parties fleurs sont éliminés par épilage chimique.

Les 340 kilogrammes de parties fleurs sont introduits dans un foulon contenant un bain composé de :
Eau froide : 170 litres
Sulfure de sodium à 60 % : 6,8 kilogrammes

Le foulon est mis en rotation pendant 45 minutes à 1 heure.

On procède ensuite à un lavage des peaux avec 340 litres d'eau froide pendant 30 minutes.

Les peaux épilées sont alors neutralisées jusqu'à pH : 6,5 dans le foulon mis en rotation pendant une heure avec un bain contenant :
Eau froide : 170 litres
Acide lactique : 3,40 kilogrammes
Métabisulfite : 0,68 kilogramme

Les peaux sont de nouveau lavées avec 340 litres d'eau froide pendant 30 minutes.

Pour finir, elles sont blanchies de manière à détruire les sulfures avec un bain contenant :
Eau froide : 340 litres
Peroxyde d'hydrogène : 6,8 kilogrammes

Les parties fleurs des peaux épilées et neutralisées à pH 6,5 sont lavées une dernière fois avec 340 litres d'eau froide pendant 30 minutes.

### Opérations mécaniques

Les peaux subissent alors une partie du traitement effectué sur les dermes, à savoir :
- prédécoupe en pièces avec la coupeuse du type "Laursen"
- découpe par passage dans la machine du type cutter "Stephan", sans ajout de glace ou d'eau.

On obtient ainsi 300 kilogrammes d'un produit assimilable à un hâchis dont le taux d'humidité est de 70 % et la taille moyenne des particules de 0,5 à 3 centimètres. Ce produit est séché sous agitation dans un séchoir du type commercialisé par la Société "Guedu"( société domiciliée en France) à vide partiel, permettant l'évaporation de l'eau à une température ne dépassant pas 35°C. En réglant les conditions de séchage pour obtenir une teneur en eau finale d'environ 20 %, on récupère 112 kilogrammes de granulat sec. Ce granulat, comme dans le cas du produit humide est constitué de petits grains possèdant encore la structure du feutrage dermique. La réduction finale en fibres sèches bien différenciées et de très faible longueur inférieure à 0,5 millimètre est effectuée dans un broyeur à sec à broches du type de celui commercialisé par la Société "Alpine" (domiciliée en RFA) dans lequel le rotor et le stator sont hérissés de palettes. On obtient ainsi 110 kilogrammes de poudre sèche à 18 % d'humidité, utilisable notamment comme ingrédient dans l'alimentation humaine et animale, ou dans le domaine des produits d'hygiène corporelle, pharmaceutiques pour la médecine humaine et vétérinaire, cosmétiques, ou bien encore dans le domaine de l'industrie textile et papetière.

### TRAITEMENT A PARTIR DE PEAUX BSH

Dans cet exemple, les matières premières sont constituées par des peaux dénommées Blanc Stabilisé Humide qui ont été soumises à un traitement de préconditionnement ou prétannage, en présence de sulfate d'alumine, après picklage en présence de résines acryliques.

Les peaux préconditionnées B.S.H non retenues pour la fabrication du cuir ou les autres dans le cas d'une faible demande du marché sont, tout d'abord, refendues en épaisseur à l'aide d'une refendeuse traditionnelle de tannerie.

A partir de 500 Kilogrammes de peaux B.S.H., on produit un lot de 175 kilogrammes de parties fleurs (1/3) et un lot de 325 kilogrammes (2/3) de parties dermes. Chacun de ces lots est prédécoupé en pièces d'environ 20 x 20 cm, avec une coupeuse du type de celle commercialisée par la Société "Laursen" avant d'être détanné ou déconditionné.

Conformément à l'invention, les parties dermes sont destinées à la fabrication de pâte humide à base de collagène, tandis que les parties fleurs sont destinées à la fabrication de poudre sèche.

Fabrication de pâte humide avec les parties dermes des peaux B.S.H.

Dans un premier temps, les 325 kilogrammes de dermes sont déconditionnés comme suit en foulon :

1er bain :

| | |
|---|---|
| Eau | 650 litres |
| NaCl | 65 kilogrammes |
| HCl | 26 kilogrammes |
| à 35 % | |

Rotation 1 heure - vidange du bain

2ème bain :

| | |
|---|---|
| Eau | 650 litres |
| NaCl | 65 Kilogrammes |
| HCl | 26 kilogrammes |
| à 35 % | |

Rotation 1 heure - vidange du bain

1er lavage :

| | |
|---|---|
| Eau | 650 litres |
| NaCl | 65 Kilogrammes |

Rotation 1/2 heure - vidange du bain

2ème lavage :

| | |
|---|---|
| Eau | 650 litres |
| NaCl | 65 kilogrammes |

Rotation 1/2 heure - vidange du bain.

Neutralisation :

| | |
|---|---|
| Eau | 330 litres |
| NaCl | 13 kilogrammes |
| Na$_2$CO$_3$ | 4,9 kilogrammes |

Rotation 2 heures - vidange du bain et des peaux.

On obtient 435 Kilogrammes de dermes déconditionnés et prédécoupés à 30 % en poids de matières sèches.

Dans un second temps, les parties dermes ainsi traitées sont mélangées à 215 kilogrammes de glace en paillettes pour alimenter la machine dite cutter vertical, du type de celle commercialisée par la Société "Stephan" qui les découpe en petits morceaux d'une taille d'environ 3 x 3 centimètres.

La glace en paillettes permet d'amener la matière sèche à une concentration de l'ordre de 20 % en poids.

Ces petits morceaux sont ensuite introduits dans un prébroyeur du type commercialisé sous la dénomination "Microcut" de la Société Stephan", dont l'organe de coupe est constitué d'une tête à six dents et d'une couronne avec lames inclinées à dix-neuf dents. Cette opération permet d'obtenir 650 kilogrammes d'une pâte très grossière destinée à alimenter le broyeur proprement dit. A ce stade, le produit obtenu est composé de tous petits morceaux de quelques millimètres mais dont chacun a conservé sa structure primitive à savoir : le réseau tridimensionnel de fibres de collagène. La dernière étape de broyage va donc consister à faire éclater cette structure.

Afin de réaliser la dernière étape sans une élévation de température au delà de 40°C, il est nécessaire d'ajouter encore 162 kilogrammes de glace en paillettes ou d'eau froide aux 650 kilogrammes précédents pour obtenir une pâte fine et humide de fibres de collagène. Le dispositif de broyage est formé par un couple rotor/stator, équipé chacun de séries de couronnes concentriques crénelées de lames tranchantes avec des inclinaisons différentes et d'un système de répartition à l'entrée de l'appareil. Un broyeur de ce type est commercialisé sous la dénomination "Trigonal" par la Société Siefer. La pâte fine et humide ainsi obtenue peut être soit utilisée immédiatement, soit conservée par le froid.

Cette pâte est donc constituée d'eau et de fibres de collagène bien différenciées. On le vérifie facilement au microscope. Sa composition chimique est la suivante :
pH 6,2 ± 0,3
Matières sèches : 16 - 18 % en poids comprenant au moins 85 % de collagène
Matières grasses: inférieur à 1% en poids
Matières minérales : inférieur à 1,5 % en poids.
Aluminium (Al III) : inférieur à 30 mg/kg.

Comme indiqué précédemment, les parties fleurs obtenues permettent la fabrication de poudre sèche de collagène.

Fabrication de poudre sèche

Dans un premier temps, les 175 kilogrammes de fleurs prè-découpées sont déconditionnées en foulon de la manière suivante :

EP 0 358 585 A1

1er bain :

Eau :                                     350 litres
NaCl :                              35 Kilogrammes
HCl :                               14 Kilogrammes
à 35 %

Rotation 1 heure - vidange du bain.

2ème bain :

Eau :                                     350 litres
NaCl :                              35 Kilogrammes
HCl :                               14 kilogrammes

Rotation 1 heure - vidange du bain.

1er lavage :

Eau :                                     350 litres
NaCl :                              35 Kilogrammes

Rotation 1/2 heure - vidange du bain.

2ème lavage :

Eau :                                     350 litres
NaCl :                              35 kilogrammes

Rotation 1/2 heure - vidange du bain.

Neutralisation :

Eau :                                     180 litres
NaCl :                                 7 kilogrammes
Na2CO3 :                            2,7 kilogrammes

Rotation 2 heures - vidange du bain et des peaux.

On obtient 235 Kilogrammes de fleurs déconditionnées et pré-découpées.

Le lot de 235 Kilogrammes de fleurs prédécoupées et déconditionnées subit une découpe dans la machine du type cutter "Stephan" sans ajout de glace ou d'eau.

On obtient ainsi un produit assimilable à un hâchis dont le taux d'humidité est de 70 % et la taille moyenne des particules de 0,5 à 2 centimètres. Ce produit est séché sous agitation dans un séchoir du type commercialisé par la Société "Guedu", à vide partiel, permettant l'évaporation de l'eau à une température ne dépassant pas 35° C. En réglant les conditions de séchage pour obtenir une teneur en eau finale d'environ 20 %, on récupère 88 kilogrammes de granulat sec. Ce granulat, comme dans le cas du produit humide est constitué de petits grains possèdant encore la structure du feutrage dermique. La réduction finale en fibres sèches bien séparées et de très faible longueur inférieure à 0,5 millimètre est effectuée dans un broyeur à sec à palettes du type de celui commercialisé par la Société "Alpine", dans lequel le rotor et le stator sont hérissés de palettes. On obtient ainsi 85 kilogrammes de poudre sèche à 18 % d'humidité, utilisable notamment comme ingrédient dans l'alimentation humaine et animale, ou dans le domaine des produits d'hygiène corporelle, pharmaceutiques pour la médecine humaine et vétérinaire, cosmétiques, ou bien encore dans le domaine de l'industrie textile et papetière.

Les produits à base de collagène obtenus par ce procédé présentent un taux de contamination tolérable pour les applications alimentaires sans traitement chimique particulier et sans emploi d'antiseptiques, sous réserve du respect de certaines conditions d'hygiène et de propreté dans la fabrication. En ce qui concerne les applications en pharmacie ou en parapharmacie, les produits peuvent être soumis à un traitement de stérilisation.

**Revendications**

1 - Procédé d'obtention de collagène natif à partir de peaux animales, selon lequel la peau de départ, retenue à titre de matière première, présente un état d'élaboration dans la filière de transformation de la peau brute ou fraîche vers le cuir, choisi parmi les états suivants, à savoir un état brut ou frais, et un état intermédiaire, dans lesquels le collagène constitutif a conservé pour l'essentiel son intégrité, et selon lequel la peau traitée est la peau de départ, ou une peau déconditionnée, selon que la peau de départ est dans un état brut ou intermédiaire respectivement, caractérisé en ce qu'on défibrille d'une façon quasi-complète la peau traitée, sans adjonction de produit chimiquement actif, au moins par les opérations successives suivantes :

a) éventuellement lavage des peaux traitées avec une solution aqueuse froide

b) refendage de la peau lavée, pour obtenir une partie derme d'une part, et une partie fleur d'autre part

c) réduction mécanique progressive de la surface et à froid de la partie fleur et/ou de la partie derme, par prédécoupage en pièces de surface réduite, puis découpage desdites pièces en morceaux plus petits, en présence d'un agent de refroidissement et de dilution prévu pour amener la matière sèche de la suspension obtenue à une concentration comprise entre 10 et 30 % en poids, et maintenir la température inférieure à environ 40° C

d) traitement postérieur par broyage, par voie sèche ou humide, de la ou des suspensions obtenues, pour détruire le feutrage dermique et obtenir au moins un produit final riche en collagène fibreux pratiquement non dénaturé.

2 - Procédé selon la revendication 1, caractérisé en ce que la réduction mécanique progressive de la surface est effectuée sur la partie derme, pour obtenir une suspension comprenant entre 10 et 30 % de matière sèche, et le traitement postérieur est effectué sur le mélange obtenu, par broyage progressif et à froid de ladite suspension, toujours en présence d'un agent de refroidissement et de dilution, pour obtenir finalement une pâte fine et humide de collagène.

3 - Procédé selon la revendication 2, caractérisé en ce que le traitement postérieur consiste en un prébroyage pour obtenir une pâte grossière, suivi par un broyage final plus fin, pour obtenir la pâte fine et humide.

4 - Procédé selon la revendication 1, caractérisé en ce que la pâte fine et humide est déshydratée, de manière à obtenir des fibres sèches de collagène, de longueur comprise entre environ 1 et 2 centimètres.

5 - Procédé selon la revendication 1, caractérisé en ce que la réduction mécanique progressive de la surface est effectuée sur la partie fleur, et le traitement postérieur est effectué sur la suspension obtenue par cette division de la partie fleur, par séchage et à une température au plus égale à 35°C de ladite suspension, pour obtenir un granulat sec, puis par réduction par broyage dudit granulat, pour obtenir finalement une poudre fine de fibres collagène.

6 - Procédé selon l'une quelconque des revendications 1 à 5, caractérisé en ce que la peau de départ est une peau fraîche, moyennant quoi la peau traitée s'identifie à cette dernière.

7 - Procédé selon les revendications 1 et 6, caractérise en ce qu'après refendage de la peau lavée, et avant réduction mécanique progressive de la surface de la partie fleur, cette dernière est soumise a un traitement chimique, limité aux follicules pileux et à l'épiderme, puis neutralisée et lavée à l'eau froide.

8 - Procédé selon l'une quelconque des revendications 1 à 6, caractérisé en ce que la peau de départ est une peau dans un état intermédiaire entre l'état brut ou frais et l'état complètement tanné (cuir), et la peau traitée est obtenue par déconditionnement de la peau de départ.

9 - Procédé selon la revendication 8, caractérise en ce que la peau de départ est une peau préconditionnée, biologiquement stabilisée, et le déconditionnement de la peau de départ s'effectue avec des agents neutralisants et bactéricides.

10 - Procédé selon la revendication 9, caractérisé en ce que le déconditionnement consiste à mettre au moins une fois les peaux de départ en agitation dans un bain d'une solution aqueuse d'acide et de sel, à les laver au moins une fois sous agitation à l'aide d'un bain d'une solution saline aqueuse, et à les neutraliser sous agitation en présence d'une solution saline et alcaline.

11 - Procédé selon la revendication 8, caractérisé en ce que le déconditionnement intervient après refendage, mais avant réduction mécanique progressive de la surface des peaux.

12 - Pâte fine et humide, obtenue à partir de peaux fraîches, riches en collagène fibreux pratiquement non dénaturé, caractérisée en ce qu'elle présente la composition chimique suivante :

- pH 6,5 ± O,5
- matières sèches : 15 à 18 % en poids, dont au moins 94 % de collagène fibreux
- matières grasses au plus égales à 0,5 % en poids
- matières minérales au plus égales à 0,1 % en poids

13 - Pâte fine et humide, obtenue à partir de peaux préconditionnées, biologiquement stabilisées, riches en collagène non dénaturé, caractérisée en ce qu'elle présente la composition chimique suivante :
- pH : 6,2 ± 0,3
- matières sèches : 15 à 20 % en poids, dont au moins 85 % de collagène fibreux
- matières grasses au plus égales à 1 % en poids
- matières minérales au plus égales à 1,5 % en poids
- aluminium au plus égal à 30 mg/kg

14 - Poudre fine, riche en collagène fibreux pratiquement non dénaturé, caractérisée en ce que les fibres de collagène sec sont séparées, et ont une longueur au plus égale à 0,5 mm, la teneur en eau de la poudre étant comprise entre 15 et 25 % en poids.

15 - Emballages alimentaires comestibles, obtenus à partir d'une pâte selon la revendication 12 ou 13, ou obtenue par le procédé selon l'une quelconque des revendications 1 à 11.

16 - Produit alimentaire destiné à l'alimentation humaine ou animale, comportant un ingrédient choisi parmi les compléments suivants, à savoir une pâte selon la revendication 12 ou 13, une poudre selon la revendication 14, et un produit obtenu finalement par le procédé selon l'une quelconque des revendications 1 à 11.

17 - Article d'hygiène corporelle du type tampon périodique, couche ou similaire, incorporant des fibres sèches de collagène obtenues selon le procédé de la revendication 4.

18 - Produit pharmaceutique pour la médecine humaine ou vétérinaire, tel que pansement hémostatique, comportant un produit choisi parmi les produits suivants, à savoir une pâte selon la revendication 12 ou 13, une poudre selon la revendication 14, et un produit obtenu par le procédé selon l'une quelconque des revendications 1 à 11.

19 - Produit cosmétique, tel que masque de beauté, comportant un produit choisi parmi les produits suivants, à savoir une pâte selon la revendication 12 ou 13, une poudre selon la revendication 14, et un produit obtenu par le procédé selon l'une des quelconque des revendications 1 à 11.

20 - Produit textile ou papetier, comportant un produit choisi parmi les produits suivants, à savoir une pâte selon la revendication 12 ou 13, une poudre selon la revendication 14, et un produit obtenu par le procédé selon l'une quelconque des revendications 1 à 11.

FIG.1

# FIG.2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | FR-A-1 568 829 (CENTRE TECHNIQUE DU CUIR)<br>* Page 4, ligne 25 - page 5, ligne 35 *<br>--- | | C 08 L 89/06<br>A 61 K 35/36<br>A 61 K 37/12 |
| A | CHEMICAL ABSTRACTS, vol. 77, no. 22, 27 novembre 1972, page 78, abstract no. 141505v, Columbus, Ohio, US; M. DUBOIS et al.: "Uses of collagen from leather waste materials", & TECHNICUIR 1972, 6(4), 60-7<br>----- | | |

**DOMAINES TECHNIQUES RECHERCHES (Int. Cl.5)**

A 61 K
C 08 L

Le présent rapport a été établi pour toutes les revendications

| Lieu de la recherche | Date d'achèvement de la recherche | Examinateur |
|---|---|---|
| LA HAYE | 26-10-1989 | TURMO Y BLANCO C.E. |